# EUROPEAN PATENT APPLICATION

(11) **EP 1 787 595 A1**
(43) Date of publication of application: **23.05.2007**
(21) Application number: 05781279.4
(22) Date of filing: 30.08.2005
(51) Int. Cl.: A61B 18/12, A61B 17/34

(54) **VARIX TREATMENT SYSTEM**

(30) Priority: 01.09.2004 JP 2004253957
(71) Applicant: JMS Co., Ltd., Hiroshima-shi, Hiroshima 730-8652 (JP)
(72) Inventor: ORIHASHI, Kazumasa;, Hiroshima-shi, Hiroshima 7340002 (JP); HAYASHI, Shuro, 7380036 (JP)
(74) Representative: Herrmann, Uwe
(86) International application number: PCT/JP2005/015733
(87) International publication number: WO 2006/025366

(57) **Abstract**

A puncturing member **2** including a puncturing part **2a** for puncturing a vein in which a varicosis is formed is formed of a conductor. A counter electrode plate **5** to be allowed to be in contact with the surface of an organism and a foot switch **18** are connected to an apparatus main body **4.** An inverter circuit for generating high frequency power is provided in the apparatus main body **4.** After the end part of a cylinder member **3** is allowed to puncture skin, the puncturing member **2** is inserted into the cylinder member **3** so that the puncturing part **2a** punctures the vein in which the varicosis is formed. The high frequency power generated at the inverter circuit is supplied to the puncturing part **2a.** Heat generated at the puncturing part **2a** cauterizes the vein to block the bloodstream, thereby degenerating the vein.

## Description

### Technical Field

The present invention relates to a treatment apparatus for treating a varicosis formed in a vein of an organism.

### Background Art

Multiple veins are distributed in dermis and hypodermal tissue which compose skin of an organism. Injury, for example, valve failure or the like may occur in a vein distributed in the skin to form a varicosis in some cases. When a varicosis is formed in a vein in the skin, the vein is liable to be raised in the surface of the skin in a webbed or reticular state to be conspicuous, which necessitates a varicosis treatment especially in cosmetic view. As one of treatment methods, a sclerotherapy disclosed in, for example, Patent Document 1 has been known conventionally. The sclerotherapy is a method in which a sclerosing agent composed of a medicine that causes injury in organic tissue is injected into a vein in which a varicosis is formed with the use of an injection syringe to cause injury in the inner wall of the vein and the components of the blood, thereby forming a thrombus. The thus formed thrombus blocks the bloodstream in the vein to harden and degenerate the vein in which the bloodstream is blocked, so that the raised vein fades away from the surface of the skin.
Patent Document 1: Japanese Patent Application Laid Open Publication No. 8-98858A (Page 2)

### Summary of the Invention

### Problems that the Invention is to Solve

The sclerosing agent used for the sclerotherapy in Patent Document 1, however, causes injury in the organic tissue, and therefore, a defense reaction of the organism is exerted excessively to cause inflammation in the vein or the organic tissue therearound. Further, the sclerosing agent after injection into the vein diffuses through the bloodstream in the vein to a part that requires no treatment, widening the inflamed part undesirably. When the inflammation is caused over a wide range in the vicinity of the surface of the skin, skin injury, such as storage disease where a pigment is deposited in the skin or the like is liable to be caused, which would result in cosmetically poor effects of the treatment. As well, because a plenty of sympathetic nerves are distributed in the skin, wide-ranged inflammation would cause ache.

The present invention has been made in view of the foregoing and has its object of performing treatment for a varicosis formed in a vein distributed in skin of which effects are cosmetically excellent and which causes less or no pain by blocking the bloodstream in the vein without using a sclerosing agent.

### Means for Solving the Problems

To attain the above object, in the present invention, a puncturing member is allowed to puncture a vein in which a varicosis is formed or organic tissue in the vicinity thereof, and electric power is supplied thereto to coagulate the vein electrically for occluding the vein.

Specifically, in a first invention, a varicosis treatment apparatus includes: a puncturing member including a puncturing part for puncturing a vein in which a varicosis is formed or an organic tissue in the vicinity of the vein, at least a part of the puncturing part being formed of a conductor; and power supply means which supplies electric power for heat generation to the part of the puncturing part which is formed of the conductor so that heat is generated at the puncturing part for cauterizing the vein around the puncturing part with the puncturing part allowed to puncture the vein in which the varicosis is formed or the organic tissue in the vicinity of the vein.

In the above arrangement, when the puncturing part of the puncturing member is allowed to puncture the vein in which the varicosis is formed and power supply by the power supply means starts, the heat is generated at the puncturing part to cauterize the vein around the puncturing part, thereby occluding the vein. As well, when the puncturing part of the puncturing member is allowed to puncture the organic tissue in the vicinity of the vein in which the varicosis is formed and power supply by the power supply means starts, the heat generated at the puncturing part cauterizes the organic tissue in the vicinity of the vein and also the vein in which the varicosis is formed, thereby occluding the vein. As a result, the bloodstream in the vein in which the varicosis is formed is blocked to cause degeneration of the vein, so that the vein fades away from the surface of the skin. Thus, the varicosis treatment without injecting a sclerosing agent is realized in contrast to the conventional one, with a result that the varicosis treatment causes less or no inflammation over a wide range of the vein and the tissue therearound.

Referring to a second invention, in the varicosis treatment apparatus of the first invention, the puncturing member is formed of a conductor in the form of a needle, the puncturing part is provided at the tip end of the puncturing member, and the power supply means is connected to the base end of the puncturing member.

In the above arrangement, the puncturing member is formed of a conductor, so that the electric power can be supplied to the puncturing part only by connecting the power supply means to the base end of the puncturing member, simplifying the structure of the puncturing member. Further, the puncturing member is in the form of a needle to alleviate the pain at puncture of the puncturing part into the skin and to suppress bleeding. In addition, the cauterized range is minimized, which means cauterization at only necessary part of the vein or the organic tissue in the vicinity thereof.

Referring to a third invention, in the second invention, a part of the puncturing member which is located on the base end side from the puncturing part is covered with an insulator for insulating skin from the puncturing member.

In the above arrangement, the skin is insulated from the puncturing member, so that no electric power is supplied from the power supply means to the skin, thereby preventing cauterization of the skin.

Referring to a fourth invention, the varicosis treatment apparatus of the second invention further includes: a cylinder member having one end from which the puncturing part of the puncturing member is inserted with the other end thereof puncturing skin.

In the above arrangement, a part of the puncturing member which is located on the base end side from the puncturing part is covered with the cylinder member to be out of contact with the skin. Accordingly, even if the heat generated at cauterization of the vein or the organic tissue in the vicinity thereof is transferred to the part of the puncturing member which is located on the base end side from the puncturing part, the heat is suppressed from being transferred to the skin.

Referring to a fifth invention, the varicosis treatment apparatus of the fourth invention further includes: suction means connected to the one end of the cylinder member.

With the above arrangement, connection of the one end of the cylinder member to the suction means enables suction of the blood in the vein after puncture of the other end of the cylinder member into the vein in which the varicosis. This enables cauterization with the vein flattened.

Referring to a sixth invention, in the varicosis treatment apparatus of any of the first to fifth inventions, an output value of the power supply means is set within a range between three watts and eight watts, both inclusive.

In the above arrangement, 3-watt or larger electric power is supplied at a part of the puncturing part which is formed of a conductor. Accordingly, electric power required for cauterizing the vein completely can be obtained at the puncturing part. Further, the thus obtained electric power is eight watts or lower, preventing the organic tissue around the vein from being cauterized undesirably.

Referring to a seventh invention, in the varicosis treatment apparatus of any of the first to sixth inventions, the power supply means includes a switch for staring power to be supplied to the part of the puncturing part which is formed of the conductor and a control circuit for allowing the power to be supplied for two seconds or shorter from operation of the switch and then stopping the supply.

In the above arrangement, operation of the switch stops power supply after supplying the power for two seconds or shorter to a part of the puncturing part which is formed of the conductor. This means no long-time continuation of power supply, thereby preventing undesirable cauterization of the organic tissue around the vein.

### Effects of the Invention

In the first invention, the puncturing part of the puncturing member is allowed to puncture the vein in which the varicosis is formed or the organic tissue in the vicinity thereof, and the electric power is supplied from the power supply means to the part of the puncturing part which is formed of the conductor for cauterizing the vein around the puncturing part. Accordingly, the varicosis treatment can be performed without using any conventional sclerosing agents. This obviates inflammation over a wide range of the vein and the organic tissue in the vicinity thereof, leading to cosmetically excellent treatment with skin injury prevented from being caused and with pain suppressed.

According to the second invention, with the puncturing member formed of the conductor in the form of a needle, cost reduction of the treatment apparatus is achieved. Further, with the needle-like puncturing member, pain and bleeding can be suppressed, and the cauterized range is minimized, which means in that only a to-be-treated part can be cauterized. Hence, low invasive treatment is achieved.

In the third invention, the insulator insulates the skin from the puncturing member, thereby preventing cauterization of an undesirable part of the skin to achieve low invasive treatment.

In the fourth invention, the puncturing part of the puncturing member is inserted into the cylinder member puncturing the skin, and therefore, the skin is prevented from being damaged by the heat at cauterization of the vein or the organic tissue in the vicinity thereof.

In the fifth invention, which includes the suction means connected to the one end of the cylinder member, the blood in the vein is sucked, thereby flattening the vein to allow the puncturing part to adhere to the inner face of the vein. This increases the efficiency of vein cauterization to reduce a time period required for occluding the vein and to occlude the vein securely, thereby blocking the bloodstream.

In the sixth invention, the electric power in the range between three watts and eight watts, both inclusive, is supplied to the part of the puncturing part which is formed of the conductor, thereby occluding the vein securely and preventing an undesirable part of the organic tissue around the vein from being cauterized.

In the seventh invention, the electric power is supplied to the puncturing part for two second or shorter by operating the switch of the power supply means, thereby reliably preventing an undesirable part of the organic tissue around the vein from being cauterized.

### Brief Description of the Drawings

[FIG. **1**] FIG. **1** is a schematic illustration showing a construction of a varicosis treatment apparatus according to the present invention.
[FIG. **2**] FIG. **2** is a partial sectional view showing a puncturing member and a cylinder member in an enlarged scale.
[FIG.**3**] FIG. **3** is a block diagram of the varicosis treatment apparatus.
[FIG. **4**] FIG. **4** is an anatomical view of skin.
[FIG. **5**] FIG. **5** presents enlarged sectional views of skin for showing a procedure for treating a varicosis formed in a vein distributed in dermis, wherein FIG. **5(a)** shows a state in which the cylinder member is allowed to puncture the skin together with a needle, FIG. **5(b)** shows a state in which the needle is pull out, and FIG. **5(c)** shows a state in which a puncturing part of the puncturing member puncture the vein.

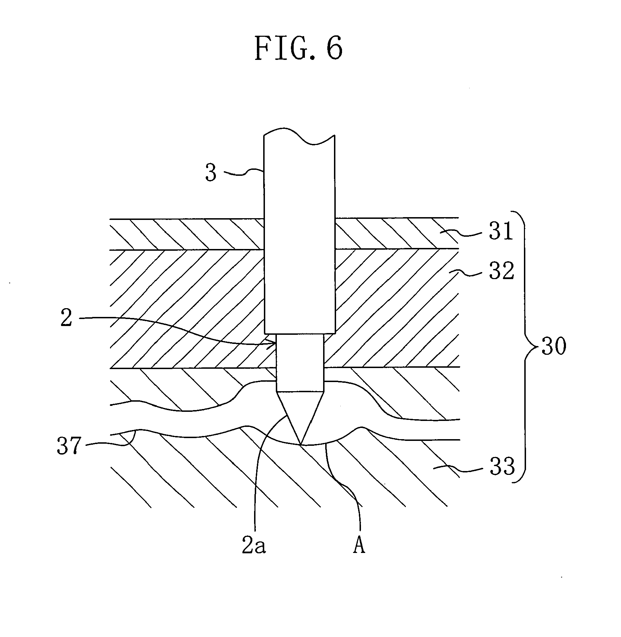

[FIG. **6**] FIG. **6** is an illustration corresponding to FIG. **5(c)** for showing a procedure for treating a varicosis formed in a vein distributed in hypodermal issue.
[FIG. **7**] FIG. **7** is a side view of a puncturing member according to Modified Example 1 of the embodiment.
[FIG. **8**] FIG. **8** is an explanatory drawing in the case where the blood in a varicosis in a vein is sucked.
[FIG. **9**] FIG. **9** is a side view of a puncturing member according to Modified Example 2 of the embodiment.
[FIG. **10**] FIG. **10** is a side view of a puncturing member according to Modified Example 3 of the embodiment.
[FIG. **11**] FIG. **11** is a side view of a puncturing member according to Modified Example 4 of the embodiment.
[FGI. **12**] FIG. **12** is a side view of a puncturing member according to Modified Example 5 of the embodiment.

### Explanation of Reference Numerals

- **1**: treatment apparatus
- **2**: puncturing member
- **2a**: puncturing part
- **3**: cylinder member
- **18**: foot switch
- **20**: control circuit
- **30**: skin
- **45**: insulator
- **50**: injection syringe (suction means)
- **A**: varicosis

### Best Mode for Carrying out the Invention

An embodiment of the present invention will be described below in detail with reference to the accompanying drawings.

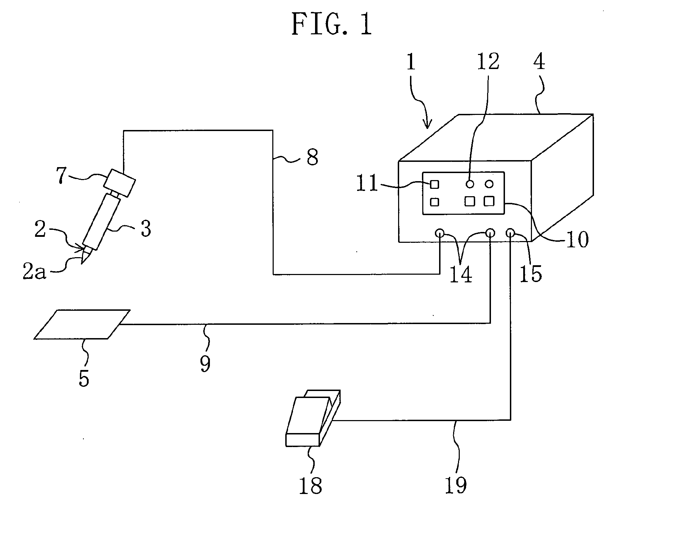

FIG. **1** shows a varicosis treatment apparatus **1** according to the embodiment of the present invention. The varicosis treatment apparatus **1** includes: a puncturing member **2** for puncturing a vein in which a varicosis is formed or organic tissue in the vicinity of the vein; a cylinder member **3** into which the puncturing member **2** is to be inserted; an apparatus main body **4** for supplying electric power to the puncturing member **2;** and a counter electrode plate **5** which is connected to the apparatus main body **4** and is to be allowed to be in contact with the surface of an organism.

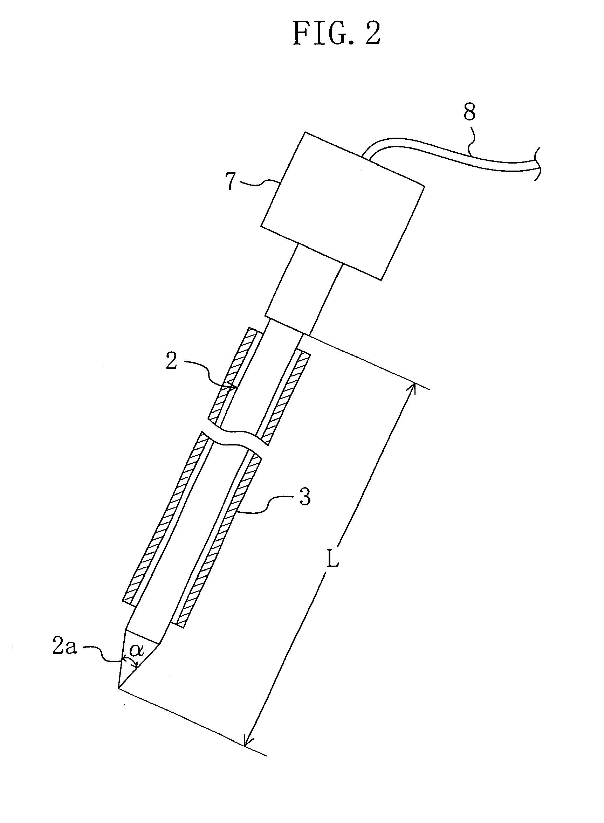

The puncturing member **2** is made of a metal material as a conductor in the form of a needle, as shown in FIG. **2,** and the tip end thereof is tapered and serves as a puncturing part **2a** to be allowed to puncture a vein. The puncturing member **2** has a length **L** in the range between 40 mm and 70 mm, both inclusive, and is arranged so as to be easily handled by the operator. The tapered angle α of the puncturing part **2a** when viewed from a side is set within the range between 25° and 35°, both inclusive, so as to be easily puncture the skin.

A gripping portion **7** to be gripped by the operator is provided at the base end of the puncturing member **2** so that the operator can smoothly manipulate the puncturing member **2.** One end of a connection cord **8** extending from the apparatus main body **4** is fixed to the gripping portion **7** so that the one end of the connection cord **8** allows the base end of the puncturing member **2** to be conductive. The counter electrode plate **5** is formed of a conductor to be conductive through one end of another connection cord **9.**

The cylinder member **3** is a generally-called metal-made indwelling needle and has an inner diameter larger than the outer diameter of the puncturing member **2** and a length shorter than the puncturing member **2.** Each end face in the longitudinal direction of the cylinder member **3** extends in the direction intersecting at a right angle with the axial line of the cylinder member **3.**

Further, as shown in FIG. **1,** there are provided at an operation panel **10** of the apparatus main body **4** a waveform selection switch **11** for selecting a waveform of output electric current between a continuous rectangular waveform and a discontinuous rectangular waveform, an output adjustment switch **12** for adjusting magnitude of the output electric current, and the like. In addition, the operation panel **10** includes terminals **14** into which the other end of the connection cord **8** for the puncturing member **2** and the other end of the connection cord **9** for the counter electrode plate **5** are plugged and another terminal **15** into which a connection cord **19** for a foot switch **18** that starts power supply to the puncturing member **2** is plugged. Power supply means of the present invention is composed of the apparatus main body **4,** the counter electrode plate **5,** the connection cords **8, 9, 19,** and the foot switch **18.**

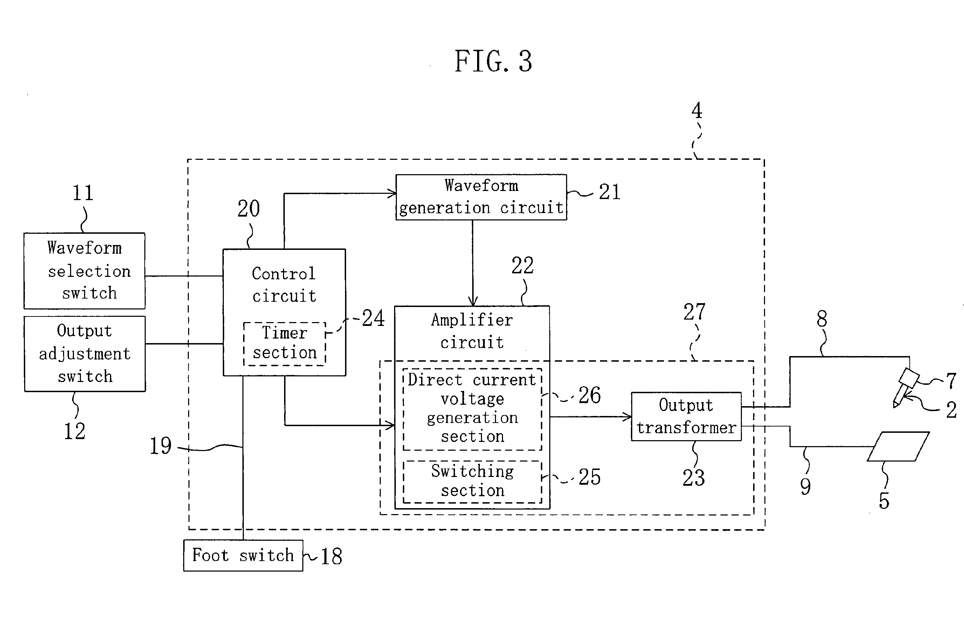

Inside the apparatus main body **4,** as shown in FIG. **3,** there are provided: a control circuit **20;** a waveform generation circuit **21** controlled by the control circuit **20** for generating a waveform of the output electric current; a variable amplifier circuit **22** for changing a waveform signal from the waveform generation circuit **21** to an output waveform; and an output transformer **23** for boosting a signal output from the variable amplifier circuit **22**

The control circuit **20** are connected to the waveform selection switch **11,** the output adjustment switch **12,** and the foot switch **18** so as to detect each operation of the switches **11, 12, 18.** Upon detection of the operation of the foot switch **18,** the control circuit **20** sends to the waveform generation circuit **21** a waveform selection signal as a drive parameter corresponding to a waveform selected by the waveform selection switch **11.** The waveform generation circuit **21** that has received the waveform selection signal generates a waveform signal corresponding to the waveform selection signal. The thus generated waveform signal is input to a switching section **25** that the variable amplifier circuit **22** includes, thereby activating the switching section **25.**

Further, the control circuit **20** includes a timer section **24** that starts its operation at the time point when the foot switch **18** is operated. While detecting, with the use of the timer section **24,** a lapse of a predetermined period from the time point when the foot switch **18** is operated, the control circuit **20** sends the waveform selection signal until the predetermined time elapses from the time point when the foot switch **18** is operated, and then, stops sending of the waveform selection signal. In other words, when the predetermined period lapses from the time point when the foot switch **18** is operated, the waveform generation circuit **21** stops sending the waveform signal so that the power supply from the apparatus main body **4** is stopped. The predetermined period set in the timer section **24** can be changed by, for example, a switch or the like provided at the operation panel **10** arbitrarily in the range of two seconds or shorter, wherein it is set to one second in the present embodiment.

Moreover, the control circuit **20** sends to a direct current voltage generation section **26** of the variable amplifier circuit **22** a voltage signal as a drive parameter corresponding to an output value set by the output adjustment switch **12.** The direct current voltage generation section **26** generates direct current voltage corresponding to the input voltage signal. The direct current voltage generated at the direct current voltage generation section **26** is applied to a primary winding (not shown) of the output transformer **23.**

The direct current voltage generation section **26,** the output transformer **23,** and the switching section **25** compose an inverter circuit **27.** In the inverter circuit **27,** when a signal from the switching section **25** and the direct current voltage from the direct current voltage generation section **26** are input to the output transformer **23,** switching operation for changing the electric current flowing in the output transformer **23** at high speed is performed to convert the electric current to high-frequency electric power. The frequency of the electric power obtained at conversion is in the range between 300 kHz and 10 MHz, and accordingly, no ventricular fibrillation of a hart is caused even if the electric power obtained at the inverter circuit **27** flows into the human body. The output adjustment switch **12** can set the high frequency electric power obtained at the inverter circuit **27** arbitrarily within the range between three watts and eight watts, both inclusive, wherein it is set to five watts in the present embodiment.

The voltage applied to the primary winding of the output transformer **23** is boosted and then is applied to a secondary winding. The secondary winding is connected to the connection cord **8** for the puncturing member **2** and the connection cord **9** for the counter electrode plate **5.** Accordingly, when the puncturing part **2a** of the puncturing member **2** is allowed to puncture the vein or the organic tissue in the vicinity of the vein and the foot switch **18** is operated with the counter electrode plate **5** allowed to be in contact with the surface of the patient's body, a secondary side circuit of the output transformer **23** is in a closed loop state, so that the high frequency electric power generated at the apparatus main body **4** flows in the loop. Since the control circuit **20** with the timer section **24** composes means for automatically stopping the power supply to the puncturing part **2a,** only one-time operation of the foot switch **18** causes the power to be supplied for one second and then to be stopped automatically.

It is noted that a switch other than the foot switch **18** may be provided for starting power supply by the switch.

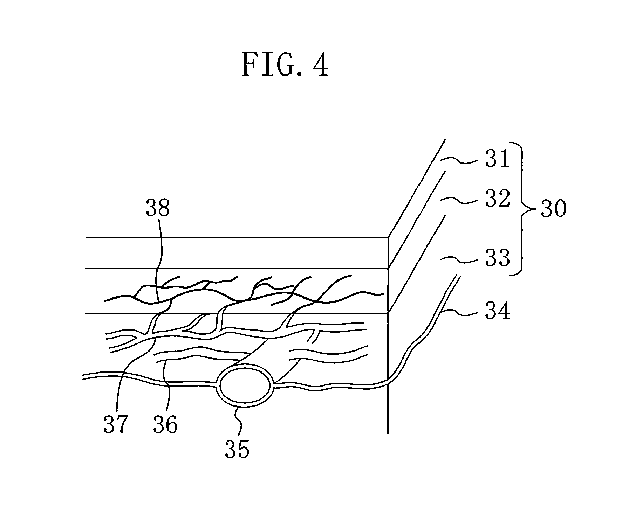

Description will be given next to a procedure for treatment of a varicosis formed in a vein distributed in human skin with the use of the varicosis treatment apparatus **1** constructed as above. Before describing the treatment procedure, veins distributed in human skin will be described with reference to FIG. **4.** The skin **30** is composed of epidermis **31,** dermis **32,** and hypodermal tissue **33,** wherein the hypodermal tissue **33** includes a fascia **32** extending along the dermis **32.** In the hypodermal tissue **33,** there are distributed a saphenous vein **35** integral with the fascia **34,** a segmental vein **36** branching from the saphenous vein **35,** and a vein **37** located on the dermis **32** side of the segmental vein **36** and formed of a small vessel. In the dermis **32,** a vein **38** formed of a vessel thinner than the vein **37** of the hypodermal tissue **33** is distributed.

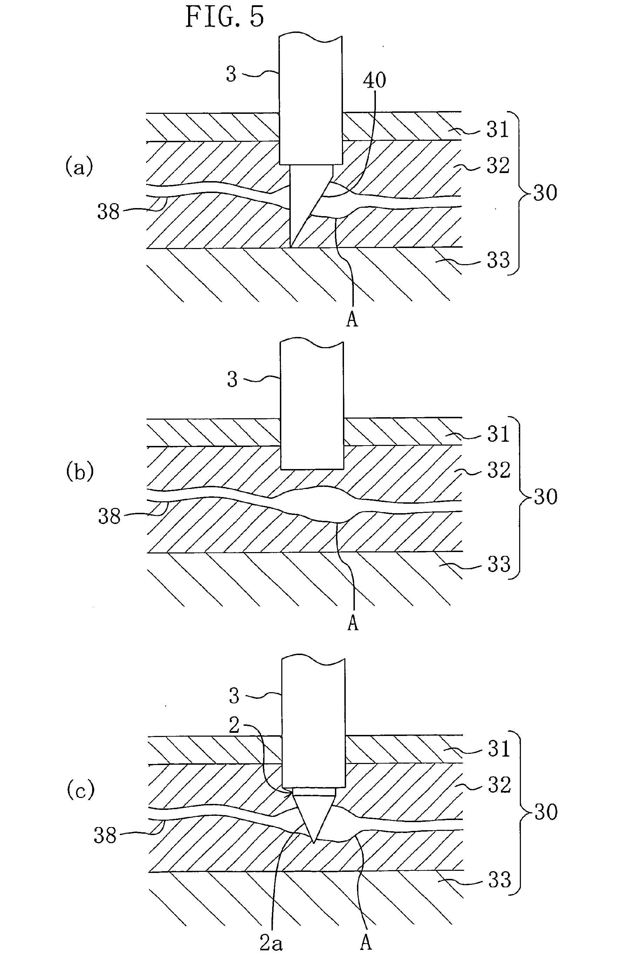

In the case where a reticular type varicosis **A** is formed in the vein **38** distributed in the dermis **32,** the cylinder member **3** is allowed to puncture first the epidermis **31** and up to a part of the dermis **32** on the epidermis **31** side, as shown in FIG. **5(a).** In this puncture, when a needle **40** is inserted into the cylinder member **3** and the cylinder member **3** is allowed to puncture the epidermis **31** and the dermis **32** with the tip end of the needle **40** protruding from the cylinder member **3,** the tip end of the needle **40** dissects the epidermis **31** and the dermis **32** so that the end part of the cylinder member **3** can puncture the epidermis **31** and the dermis **32** effortlessly. The end part of the cylinder member **3** is allowed to puncture the dermis **32** until it reaches a point in the vicinity of the varicosis A in the dermis **32,** and then, the needle **40** is pulled out from the cylinder member **3,** as shown in FIG. **5(b).**

Subsequently, as shown in FIG. **5(c),** the puncturing member **2** is inserted into the cylinder member **3** to allow the puncturing part **2a** of the puncturing member **2** to puncture the vein **38** in which the varicosis **A** is formed. Since the end part of the cylinder member **3** is positioned in the vicinity of the varicosis **A,** the puncturing part **2a** is guided to the vicinity of the varicosis **A** only by inserting the puncturing member **2** into the cylinder member **3.** Before or after puncturing the vein **38** by the puncturing member **2,** the counter electrode plate **5** is allowed to be in contact with the surface of the patient's body.

Thereafter, the waveform selection switch **11** and the output adjustment switch **12** of the apparatus main body **4** are operated to select a desired waveform and set a desired output, respectively. When the foot switch **8** is operated then, the electric power is supplied for only one second to the puncturing member **2** from the apparatus main body **4.** The electric current flowing during the period generates heat at the puncturing part **2a,** so that vein **38** around the puncturing part **2a** is cauterized by the heat generated at the puncturing part **2a.** Cauterization of the vein **38** causes the inner wall of the vein **38** to adhere and to be occluded. If the cauterization is insufficient, the foot switch **18** is operated again to supply the electric power only for one second for cauterizing the vein **38.** The above operation is repeated until the inner wall of the vein **38** adheres completely. In this way, the vein **38** in which the bloodstream is blocked is degenerated and then disappears in due course.

Referring to the aforementioned reticular type varicosis A, multiple veins **38** in which varicoses are formed are located close to each other. Therefore, in order to treat the reticular type varicosis **A,** after one of the veins **38** is cauterized as above, the other veins **38** are cauterized as well one by one. With the puncturing member **2** having an outer diameter smaller than the inner diameter of the cylinder member **3,** the other veins **38** can be cauterized only by moving the puncturing part **2** within the skin **30** with the cylinder member **3** remaining puncturing the skin **30.**

In order to treat a varicosis A formed in the vein **37** distributed in the hypodermal tissue **33** deeper than the dermis **32,** the cylinder member **3** is allowed to puncture a part deep in the dermis **32,** and then, the puncturing member **2** is inserted into the cylinder member **3** so as to allow the puncturing part **2a** to puncture the vein **37** for cauterizing the vein **37** as in the aforementioned manner.

Thus, according to the varicosis treatment apparatus **1** of the present embodiment, the varicosis A is treated by cauterizing the vein **37** or **38** without using the conventional sclerosing agent. This prevents inflammation of the vein **37** or **38** and the organic tissue around the vein **37** or **38** from spreading over a wide range. Accordingly, cosmetically excellent treatment can be achieved with skin injury prevented and with pain suppressed.

With the puncturing member **2** formed of a conductor in the form of a needle, only connection of the base end of the puncturing member **2** to the apparatus main body **4** attains power supply to the puncturing part **2a.** This lower the cost of the puncturing member **2** to lead to cost reduction of the treatment apparatus **1.** Further, with the needle-like puncturing part **2a,** pain and bleeding, which would have been accompanied by the treatment, can be suppressed, and the cauterized range is minimized with only a to-be-cauterized part cauterized. Hence, low invasive treatment can be achieved.

Moreover, the vein **37** or **38** is cauterized under a state that the puncturing member **2** is inserted into the cylinder member **3** puncturing in the skin **30** so that only the puncturing part **2a** protrudes from the end of the cylinder member **3** with a part of the puncturing member **2** which is located on the base end side from the puncturing part **2a** covered with the cylinder member **3.** Accordingly, even if the heat generated at cauterization is transferred to a part of the puncturing member **2** which is located on the base end side from the puncturing part **2a,** the heat is prevented from being transferred directly to the epidermis **31** and the organic tissue therearound. Thus, the epidermis **31** and the tissue therearound can be prevented from damaged by the heat at cauterization.

Since the electric power output from the apparatus main body **4** is set at three watts or higher, electric power sufficient for cauterizing the vein **37** or **38** can be supplied to the puncturing member **2,** resulting in reliable occlusion of the vein **37** or **38.** Further, with the electric power output from the apparatus main body **4** is set at eight watts or lower, undesirable cauterization of the organic tissue around the vein **37** or **38** is prevented, leading to low invasive treatment.

One-time operation of the foot switch **18** causes the electric power to be supplied for only one second to the puncturing part **2a,** which means no continuous power supply for a long period of time. Hence, undesirable cauterization of the organic tissue around the vein **37** or **38** is prevented, leading to low invasive treatment as well. Two seconds or shorter time period for power supply through the operation of the foot switch **18** achieves equivalent effects.

It is noted that though the puncturing part **2a** is allowed to puncture the vein **37** or **38** for directly cauterizing the vein **37** or **38** in the above embodiment, the vein **37** or **38** may be cauterized indirectly by allowing the puncturing part **2a** to puncture the organic tissue in the vicinity of the vein **37** or **38,** that is, the skin **30** for cauterizing the vein **37** or **38.** Heat used for cauterizing the organic tissue in the vicinity of the vein **37** or **38** is transferred to the vein **37** or **38** to cauterize and occlude the vein **37** or **38.**

Further, the cylinder member **3** may be made of a material having an insulation characteristic, such as resin or the like. This prevents reliably the electric current flowing in the puncturing member **2** from flowing into the skin **30.** In the case where the cylinder member **2** is made of resin, the heat at the puncturing part **2a** of the puncturing member **2** is prevented reliably from being transferred to the skin **30** when compared with the cylinder member made of metal.

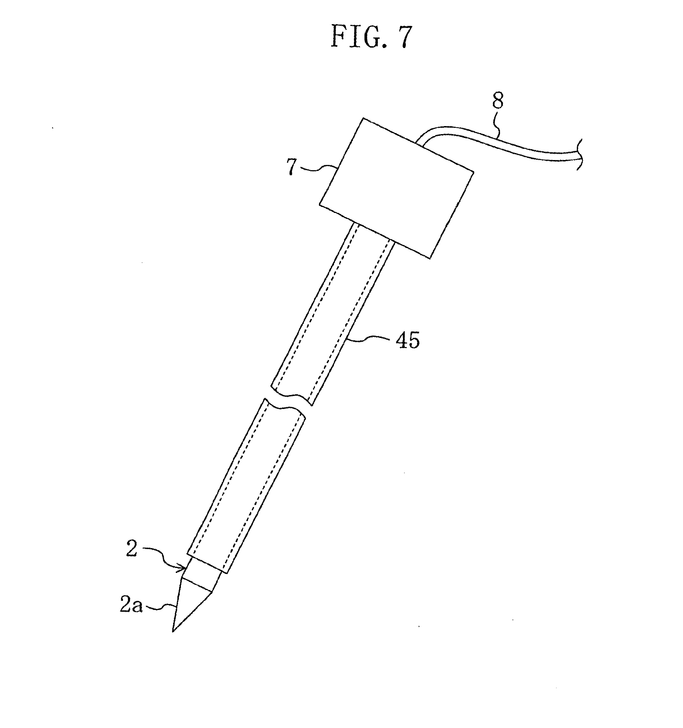

Furthermore, though the above embodiment uses the cylinder member **3,** the treatment for the varicosis **A** can be performed without using the cylinder member **3,** as in Modified Example shown in FIG. **7.** In this case, a part of the puncturing member **2** which is located on the base side from the puncturing part **2a** is covered with an insulator **45.** The insulator **45** insulates the skin **30** from a part of the puncturing member **2** which is other than the puncturing part **2a** to prevent the electric current from the apparatus main body **1** to flow into the skin **30,** preventing the skin **30** from being cauterized. Thus, lower invasive treatment can be achieved.

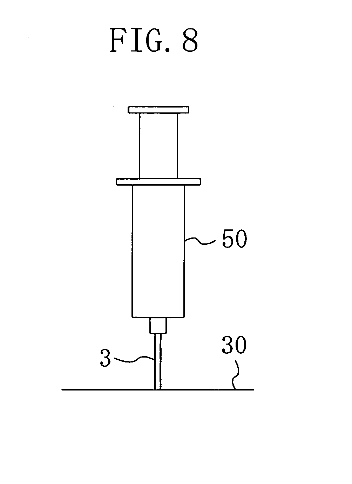

Optionally, the blood in the vein **37** or **38** may be sucked before cauterization of the vein **37** or **38.** To do so, an injection syringe **50** as suction means is attached to the end on opposite side of the cylinder member **3** from the puncturing part **2a,** as shown in FIG. **8.** The injection syringe **50** is also a component of the treatment apparatus **1.** Manipulation of the injection syringe **50** in a sucking direction with the cylinder member **3** puncturing the vein **37** or **38** causes the blood in the vein **37** or **38** to be sucked into the cylinder of the injection syringe **50.** This flattens the vein **37** or **38** and less or no blood remains in the vein **37** or **38,** allowing the puncturing part **2a** to adhere to the inner face of the vein **37** or **38.** Accordingly, the puncturing part **2a** cauterizes effectively the vein **37** or **38,** and the vein **37** or **38** is occluded surely within a shortened period of time for cauterizing the vein **37** or **38.** Hence, further lower invasive treatment can be achieved. The suction means in this case is not limited to the injection syringe **50** and any sucking apparatus such as an blood aspirator used in general medical worksites may be used.

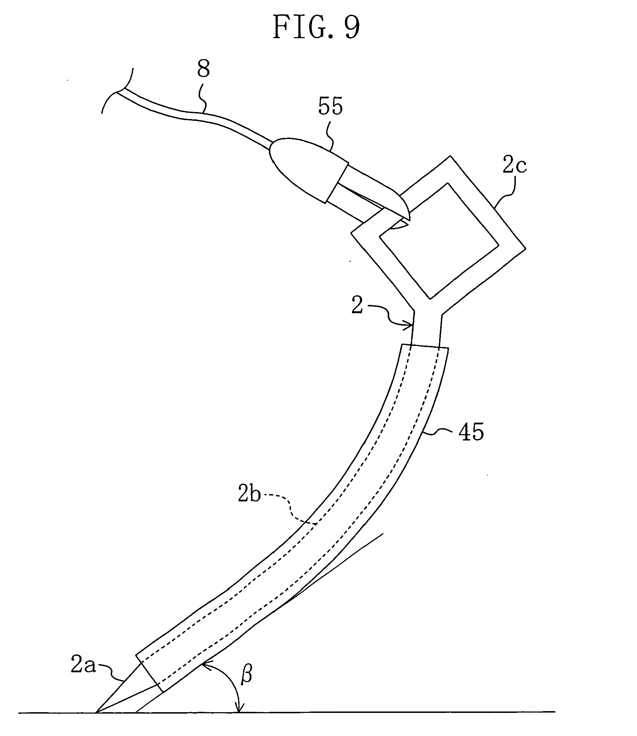

Further optionally, a curved portion **2b** may be formed in the middle in the longitudinal direction of the puncturing member **2,** as in Modified Example 2 shown in FIG. **9.** The curved portion **2b** is so formed that the inclination angle β at the tip end part of the puncturing member **2** with respect to the horizontal plane is in the range between 30° and 45°, both inclusive, in the state where the base end part of the puncturing member **2** is set substantially perpendicular. The puncturing member **2** is obtained by processing a solid bar having a diameter substantially the same as a medical injection syringe 30G (gate) to 26G and a total length of approximately 60 mm. Further, the curved portion **2b** is so formed that the puncturing member **2** has a length in the vertical direction in the range between 40 mm and 50 mm, both inclusive, in the state where the base end part of the puncturing member 2 is set substantially perpendicularly.

A part of the puncturing member **2** which is located on the base end side from the puncturing part **2a** is covered with the insulator **45** made of resin, similarly to that in Modified Example 1, with the base end part of the puncturing member **2** exposed. A rectangular frame **2c** is provided at the base end of the puncturing member **2,** wherein the frame **2c** is exposed from the insulator **45** as well. Further, in Modified Example 2, an alligator clip **55** is provided at the tip end of the connection cord **8** extending from the operation panel **10** of the apparatus main body **4.** When the alligator clip **55** clips the frame **2c** of the puncturing member **2,** the connection cord **8** is connected electrically to the puncturing member **2.** The alligator clip **55** for connecting the puncturing member **2** to the connection cord **8** facilitates attachment and detachment of the connection cord **8** to and from the puncturing member **2.** This facilitates an operation for cleaning and disinfection the puncturing member **2** detached from the connection cord **8** after treatment and an operation for exchanging puncturing members **2** according to the symptom of the varicosis **A.**

In Modified Example 2, the varicosis **A** is occluded by cauterizing the organic tissue in the vicinity of the varicosis **A.** Specifically, Modified Example 2 is applied to treatment of a web type varicosis formed in the thin vein **38** distributed in the upper part of the skin **30.** The varicosis A of the web type ranges wide in a direction along the surface of the skin **30** and the vein **38** is thin, which involves difficulty in puncturing the vein **38** by the puncturing part **2a.** In order to tackle this difficulty, the apparatus **1** of Modified Example 2 is used in such a manner that the puncturing member **2** is allowed to puncture the skin **30;** when the puncturing part **2a** reaches the vicinity of the varicosis **A,** the vein **38** is cauterized by the heat used for cauterizing the skin **30** as the organic tissue; and then the puncturing part **2a** is pushed in a direction that the varicosis A spreads, that is, the direction along the surface of the skin **30** for cauterizing the other part of the vein **38.** Further, when the puncturing member **2** is moved along the surface of the skin **30** so that the direction of the puncturing part **2a** agrees with the longitudinal direction of the vein **38,** the heat generated at the puncturing part **2a** can be transferred to a comparatively wide range of the vein **38** to cauterize the wide range of the vein **38.**

For pushing the puncturing part **2a** along the surface of the skin **30** as described above, it is necessary to set the tip end part of the puncturing member **2** in the direction along the surface of the skin **30.** When the tip end part of the puncturing member **2** is set in the direction along the surface of the skin **30,** the base end part of the puncturing member **2,** which includes the curved portion **2b,** sticks out from the surface of the skin **30** almost perpendicularly to the skin **30** and is separated from the surface of the skin **30.** Accordingly, the operator can hold the base end part of the puncturing member **2** easily, facilitating manipulation of the puncturing member **2,** as described above, for cauterizing a wide range of the vein **38.** Further, when the operator holds the base end part of the puncturing member **2** so that the tip end thereof is set substantially perpendicular to the skin **30,** resistance in puncturing the skin **30** by the puncturing member **2** is reduced.

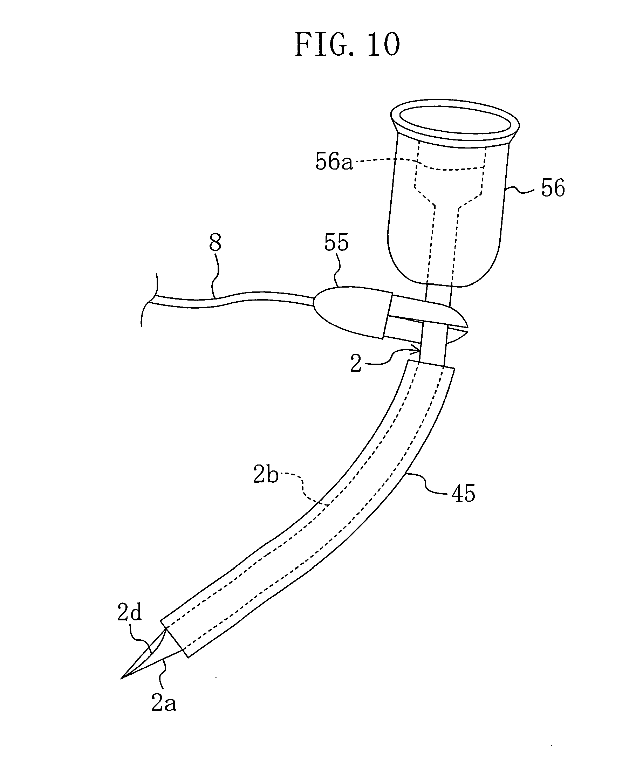

Still optionally, as in Modified Example 3 shown in FIG. **10,** the puncturing member **2** may be so hollowed that both ends thereof in the longitudinal direction are opened. Similarly to that of Modified Example 2, the puncturing member 2 includes the curved portion **2b** and is covered with the insulator **45** so that the base end part of the puncturing member **2** which is not covered with the insulator **45** is clipped by the alligator clip **55.** The puncturing part **2** is obtained by processing a hollowed bar having a diameter substantially the same as a medical injection syringe 22G or 21G and a total length of approximately 60 mm. The curved portion **2c** is so formed that the puncturing member **2** has a length in the vertical direction in the range between 40 mm and 50 mm, both inclusive, in the state where the base end part of the puncturing member **2** is set substantially perpendicularly.

In Modified Example 3, the outer face of the puncturing part **2a** forms an opening at the tip end of the puncturing member 2 and serves as a suction port **2d** for sucking the blood in the vein **37** or **38.** On the other hand, at the base end of the puncturing member **2,** a joint member **56** is mounted for joining an injection syringe (not shown). The joint member **56** is in the form of a cylinder as a whole, the inside of which communicates with the other opening at the base end of the puncturing member **2.** A tapered face **56a** with an inclination of approximately 6/100 is formed at the inner face of the joint member **56** so that the tip end part of the injection syringe is fitted therein and joined to the puncturing member **2.** In the state where the injection syringe is joined to the puncturing member **2,** the inside of the puncturing member **2** and the inside of the injection syringe communicate with each other.

In Modified Example 3, the vein **37** or **38** in which the varicosis **A** is formed is cauterized to be occluded. In detail, the blood in the varicosis **A** is sucked through the suction port **2d** by manipulating the injection syringe after puncturing the varicosis **A** by the puncturing part **2a** of the puncturing member **2,** and then, the vein **37** or **38** is cauterized directly. In direct cauterization of the vein **37** or **38,** the puncturing part **2a** can adhere to the inner face of the vein **37** or **38** with less or no blood remaining in the varicosis **A,** thereby achieving effective cauterization of the vein **37** or **38** by the puncturing part **2a.** As a result, the vein **37** or **38** is occluded surely within a shortened time period for cauterizing the vein **37** or **38,** achieving further lower invasive treatment. The joint member **56** may be joined to any suction means such as a blood aspirator used in general medical worksites rather than the injection syringe.

In Modified Example 3, similarly to Modified Example 2, the operator can hold easily the base end part of the puncturing member **2** for pushing the puncturing part **2a** of the puncturing member **2** in the direction that the varicosis **A** spreads, facilitating manipulation of the puncturing member **2.** Further, when the tip end of the puncturing member **2** is set to be substantially perpendicular to the skin for allowing the puncturing member **2** to puncture the skin **30,** resistance in puncture can be reduced.

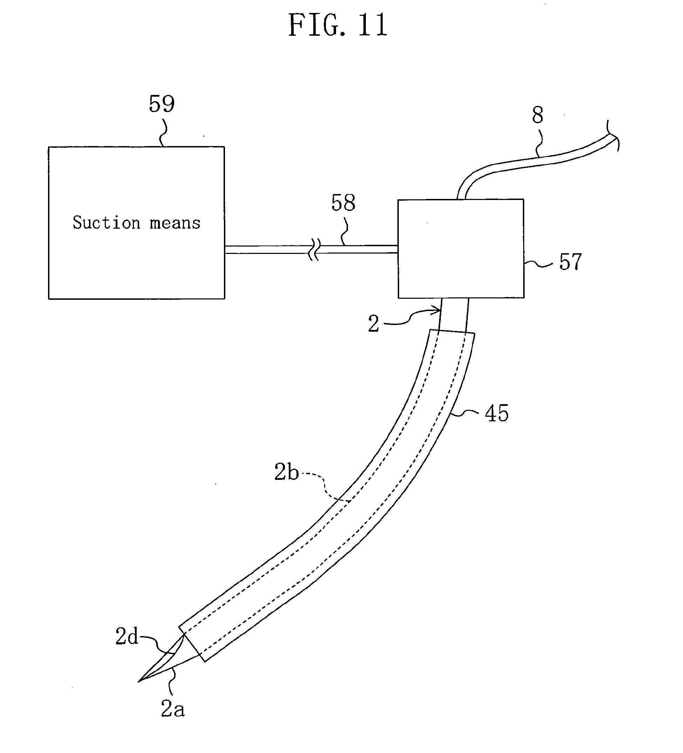

Alternatively, a connection block **57** for connecting the suction means **59** and the connection cord **8** to the base end of the puncturing member **2** may be provided as in Modified Example **4** shown in FIG. **11.** The puncturing member **2** of Modified Example 4 is structured just the same as the puncturing member **2** of Modified Example 3. The connection block **57** is provided with a connector (not shown) conductive to the base end of the puncturing member **2,** and the connection cord **8** is connected to the connector. Further, a suction hose **58** is mounted at the connection block **57** for communicating with the opening at the base end of the puncturing member **2** so that the inside of the puncturing member **2** and the suction means **59** communicate with each other through the suction hose **58.** In Modified Example 4, similarly to Modified Example 3, the vein **37** or **38** is cauterized after the blood in the vein **37** or **38** in which the varicosis A is formed is sucked.

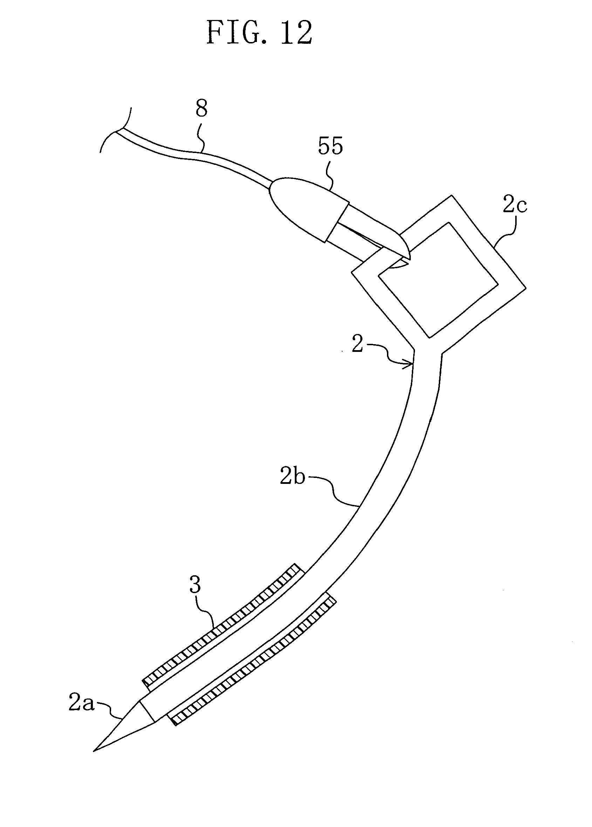

Still alternatively, as in Modified Example 5 shown in FIG. **12,** a cylinder member **3** may be provided into which the puncturing member **2** including the curved portion **2b** is to be inserted. When the cylinder member **3** is made of resin, such as Teflon (trademark), or the like, for example, the electric current flowing in the puncturing member **2** is prevented from flowing into the skin **30** and the heat at the puncturing part **2a** of the puncturing member **2** is prevented reliably from being transferred to the skin **30.**

In addition, with the use of the treatment apparatus **1,** any varicosis formed in the saphenous vein **35** or the segmental vein **36** in the skin **30** can be treated.

### Industrial Applicability

As described above, the varicosis treatment apparatus according to the present invention can be used for treatment of a varicosis formed in a vein distributed in organic skin, for example.

## Claims

1. A varicosis treatment apparatus comprising:
a puncturing member including a puncturing part for puncturing a vein in which a varicosis is formed or an organic tissue in the vicinity of the vein, at least a part of the puncturing part being formed of a conductor; and
power supply means which supplies electric power for heat generation to the part of the puncturing part which is formed of the conductor so that heat is generated at the puncturing part for cauterizing the vein around the puncturing part with the puncturing part allowed to puncture the vein in which the varicosis is formed or the organic tissue in the vicinity of the vein.

2. The varicosis treatment apparatus of Claim 1,
wherein the puncturing member is formed of a conductor in the form of a needle, the puncturing part is provided at the tip end of the puncturing member, and the power supply means is connected to the base end of the puncturing member.

3. The varicosis treatment apparatus of Claim 2,
wherein a part of the puncturing member which is located on the base end side from the puncturing part is covered with an insulator for insulating skin from the puncturing member.

4. The varicosis treatment apparatus of Claim 2, further comprising:
a cylinder member having one end from which the puncturing part of the puncturing member is inserted with the other end thereof puncturing skin.

5. The varicosis treatment apparatus of Claim 4, further comprising:
suction means connected to the one end of the cylinder member.

6. The varicosis treatment apparatus of any one of Claims 1 to 5,
wherein an output value of the power supply means is set within a range between three watts and eight watts, both inclusive.

7. The varicosis treatment apparatus of any one of Claims 1 to 6,
wherein the power supply means includes a switch for staring power to be supplied to the part of the puncturing part which is formed of the conductor and a control circuit for allowing the power to be supplied for two seconds or shorter from operation of the switch and then stopping the supply.
